(19) Europäisches Patentamt
European Patent Office
Office européen des brevets

(11) Publication number: **0 122 969**
**B1**

# (12) EUROPEAN PATENT SPECIFICATION

(45) Date of publication of patent specification: **31.05.89**

(21) Application number: **83112555.4**

(22) Date of filing: **14.12.83**

(51) Int. Cl.[4]: **C 07 K 1/00,** C 07 K 1/14, A 61 K 47/00, B 01 D 15/08, C 07 G 11/00, G 01 N 33/48, G 01 N 33/50, G 01 N 33/567, C 12 N 11/00, C 12 Q 1/04

## (54) Immobilized oligopeptides.

(30) Priority: **25.04.83 GB 8311136**

(43) Date of publication of application: **31.10.84 Bulletin 84/44**

(45) Publication of the grant of the patent: **31.05.89 Bulletin 89/22**

(84) Designated Contracting States: **AT BE CH DE FR GB IT LI LU NL SE**

(56) References cited:
**US-A-3 947 352**
**FEMS MICROBIOLOGY LETTERS, vol. 9, 1980 M.A. DE PEDRO et al. "Affinity chromatography of murein precursors on vancomycin-sepharose" pages 215-217**
**JOURNAL OF THE AMERICAN CHEMICAL SOCIETY, vol. 105, 1983 D. H. WILLIAMS et al. "Detailed Binding Sites of the Antibiotics Vancomycin and Ristocetin A: Determination of Intermolecular Distances in Antibiotic/Substrate Complexes by Use of the Time-Dependent NOE" pages 1332-1339**

(73) Proprietor: **GRUPPO LEPETIT S.P.A.
23, Via G. Murat
I-20159 Milano (IT)**

(72) Inventor: **Cassani, Giovanni
14, Via Roma
Pavia (IT)**
Inventor: **Corti, Angelo
2, Via di Vittime di Piazza della Loggia
Curnasco (BG) (IT)**
Inventor: **Riva, Ernesto
119 Corso di Porta Romana
Milano (IT)**
Inventor: **Soffientini, Adolfo
168 Via Fratelli di Dio
Sesto San Giovanni (MI) (IT)**
Inventor: **Parenti, Francesco
8, Via Emilio De Marchi
Milano (IT)**

(56) References cited:
**AFFINITY CHROMATOGRAPHY, 1974, John Wiley & Sons, London, Chapter V C. R. LOWE AND P.D.G. DEAN: "The Chemistry of Affinity Chromatography" pages 200-221**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 969 B1

(56) References cited:
The Merck Index, 9th Ed. pp. 1274-5
Medicinal Chemistry 3rd Edn. (A. Burger), p. 307

Proc. Nat. Acad. Sci. USA, vol. 69 (1972), pp. 3751-3755

Proc. Nat. Acad. Sci. USA, vol. 61 (1968), pp. 667-673

Ew. J. Biochem. vol. 117, (1981), pp. 87-91
The Molecular Basis of Antibiotic Action, 2nd, Edn. pp. 86, 87, 90, 91, 166, 167.

Journal of Chromatography Library, volume 12, "Affinity Chromatography", pp. 53-66,225,245,280 and 281.

Chem. Abs. 96:48061e

Chem. Abs. 93: 234017h

Experentia vol. 35, (1975), pages 1148-9
Antimicrobial agents and chemie therapy (1979), pp. 229-234

New Comprehensive Biochemistry vol. 8 (Separation methods) (1984), pp. 321,322,348-354

Kirk-Othmer Encyclopedia of Chemical Technology, vol. 6, pp. 35-41 and 48-52

## Description

The present invention is directed to a new modified matrix characterized in that an oligopeptide chain is linked, directly or by means of "spacer arms", to a suitable water insoluble carrier.

The new modified matrix can be represented by the following formula:

A—B—R—CO—D-Ala—Z

wherein A represents a water-insoluble carrier, B represents a bond, a spacer arm, or a polyaminoacidic residue, R represents a straight or branched ($C_1$—$C_{12}$)alkylene substituted amino or oxy group of formulae:

—HN—($C_1$—$C_{12}$)alkylene and —O—($C_1$—$C_{12}$)alkylene

respectively, and preferably a straight or branched ($C_2$—$C_6$)alkylene substituted amino or oxy group of formulae:

—HN—($C_2$—$C_6$)alkylene and —O—($C_2$—$C_6$)alkylene, respectively,

or R—CO represents a L-lysinyl residue, or a bond or a —$OC(CH_2)_nCO$— group wherein n is an integer from 1 to 6 inclusive, and preferably 2, 3, or 4; Z represents an aminoacidic residue selected from D-alanyl, glycinyl, and D-leucinyl; and D-Ala represents a D-alanyl group; with the proviso that when R—CO represents a bond B is a group having a carboxy terminal function that links the amino function of the D-alanine, or in the case both RCO and B represent a bond, A is a water-insoluble carrier having a carboxy terminal function that links the amino function of the D-alanine through a peptidic bond. In the framework of the present invention, the term "water insoluble carrier" represents any water-insoluble carrier or matrix which can be bonded to a —RCO—D-Ala—Z— group, hereinafter, "the ligand" or "Ⓛ", directly or through a spacer arm.

The term "modified matrix" generally indicates the immobilized oligopeptide of the invention, while the term "matrix" *per se* is used as a synonymous for "water-insoluble carrier".

It was found that one of the critical parameters in the modified matrix of the present invention is the specificity of the ligand for the substrate, (i.e. the selectivity of the target antibiotic for the immobilized peptide), while the *matrix "as such"* has a non-critical rôle.

The carrier must be insoluble in the solvents and buffers to be employed, it must be mechanically and chemically stable with good flow properties, it must be easily coupled to the ligand or to the "spacer arm" onto which the ligand may be attached, and it should have a large surface area accessible to the substrate to be adsorbed. Said matrices include single composition matrices such as agarose, glass, cellulose, and the like, and dual-composition or chemically modified matrices, such as agarose-coated polyacrylamide, polyacrylic coated iron particles, glycidoxy-coated glasses and the like.

Examples of water-insoluble carriers are those having hydroxy, amine or carbonyl groups or halogen atoms. Examples of water-insoluble carriers having hydroxy groups are polysaccharides (e.g. cellulose agarose, cross-linked dextran, an the like); hydroxyalkylpolystyrene resins (e.g. hydroxyalkylated styrene - divinylbenzene copolymer, and the like); polyvinylalcohols or the like. Examples of the water insoluble carriers having an amino group are aminoalkylpolysaccharides (e.g. aminoalkylcellulose, such as aminoethylcellulose or aminohexylcellulose, aminoalkylagarose such as aminohexylagarose, and the like), p - aminobenzylpolysaccharides (e.g. p - aminobenzylcellulose, p - aminobenzylagarose, and the like), chitosan, aminoalkylpolystyrene resins (e.g., aminoalkylated styrene - divinylbenzene copolymer), polyacrylamides, and aminoalkylpolyacrylamides (e.g. aminoethylpolyacrylamide, and the like), and aminoalkyl - porous glasses (e.g. aminopropyl - porous glass, and the like). Examples of water-insoluble carriers having a carboxyl group are carboxyalkylpolysaccharides (e.g. carboxyalkylagarose such as carboxyhexylagarose or carboxypentylagarose, carboxyalkylcellulose such as carboxymethylcellulose, carboxyalkyl - cross - linked dextran such as carboxymethyl - cross - linked dextran, and the like), carboxyalkylpolyacrylamides (e.g. carboxymethylpolyacrylamide, and the like), and carboxylic acid resins (e.g. acrylic acid-divinylbenzene copolymer, and the like). Examples of the water insoluble carriers having a halogen atom are halogenoalkylpolystyrene resins (e.g. chloromethylated styrene - divinylbenzene copolymer). When a halogenoalkylpolystyrene is used, it can be used as it is or it can be converted into a more activated form. For example, a halogenoalkylpolystyrene resin can be converted into a dialkylthioalkylpolystyrene resin having activity higher than that of the halogenoalkylpolystyrene resin by reacting the resin with dialkyl sulfide.

When the water-insoluble carrier possesses functional groups that can directly link to the ligand, the ligand can be directly linked to the matrix, (i.e. in the above formula, B represents a bond), however, it is in some instances preferred to link the matrix to the ligand through a spacer arm which is interposed between the carrier and the ligand to facilitate their effective binding. Representative examples of such spacer arms are the residues of formulae:

—HN—$(CH_2)_m$—NH—; —$OC(CH_2)_mCO$—; —$HN(CH_2)_mCO$—, or —$HN(CH_2)_mO$—,

wherein m represents an integer from 1 to 12 inclusive; preferably from 2 to 6, and most preferably 4.

The "polyaminoacidic residue", which is one of the possible meanings for B, represents a sequence of aminoacidic groups linked each other through a peptidic bond (—CO—NH—). The aminoacid can be one of the known alpha-aminoacids, and the average number of aminoacid units per chain is from about 2 to about 15 or more and preferably from 2 to 6. Most preferably, this polyaminoacidic residue is a poly(D-Ala) residue of (2—15) D-Ala units.

Examples of straight or branched ($C_1$—$C_{12}$)alkylene groups are ($C_1$—$C_{12}$)methylene groups, or ($C_1$—$C_{12}$)methylene groups wherein a hydrogen atom of one or more of the methylene groups is substituted by a lower alkyl group such as methyl, ethyl, propyl and the like.

Preferred embodiments of the invention are represented by those modified matrix of the invention wherein the matrix (i.e. the water-insoluble carrier) is a cross-linked dextran, cellulose or carboxymethylcellulose, controlled pore glass or derivatized glass (e.g. silanized or glycidoxy controlled pore glass, long chain alkylamine glass, aminopropyl glass, carboxyl glass, and the like), agarose, or derivatized agarose (e.g. N-hydroxysuccinimide active esters of cross-linked agarose, carboxy terminal agarose, amino terminal agarose, and the like), or polyacrylamide gels such as aminoethylpolyacrylamide which have functions that can link to free amino groups [i.e., functional derivatives having oxirane

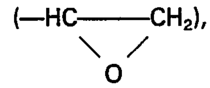

carboxy ($COOR^1$, wherein $R^1$ represents hydrogen, ($C_1$—$C_3$)alkyl, chloro, bromo or amino), halogen (chloro or bromo) and similar groups]. The spacer arm, when present, is $H_2N—(CH_2)_mCOOH$, wherein m is as above, and the oligopeptidic ligand is a D-alanyl-D-alanine dipeptide bonded to the carrier by means of a peptidic bond between the carboxy terminal function of a functionalized carrier and the free amino group of the dipeptide, or the ligand is poly(D-alanine) containing an average number of units of about 2 to 15.

A further preferred embodiment of the invention is a modified matrix wherein the carrier is carboxymethylcellulose, cellulose, porous glass, controlled pore glass, cross-linked dextrans, agarose, carboxymethylagarose, the oligopeptidic ligand is poly(D-alanine) and the bond between the carrier and the ligand is obtained by means of cyanogen bromide, epichlorohydrin, 1,4 - bis(2,3 - diepoxypropoxy)-butane or 3 - aminopropyltriethoxysilane.

Background of the invention

It is known that some antibiotics act by interfering with the synthesis of the bacterial cell-wall. More particularly, *penicillins, cephalosporins* and other beta-lactams prevent the maturation of the peptidoglycan by inhibiting the cross-linking reaction trough inhibition of transpeptidases and carboxypeptidases; bacitracin A blocks the phosphorylase that liberates one of the two terminal phosphates from the lipid carrier involved in the transport of the muramylpentapeptide through the cell membrane from the cytoplasm to the outside; as a consequence of this blocking the lipid carrier can no longer function as an acceptor of muramylpentapeptide (see G. Lancini. F. Parenti, "The antibiotics", Springer-Verlag, N.Y., 1982, pp. 42—48). *Vanomycin* and *Ristocetin A* interfere with the peptidoglycan which is the main structural component of bacterial cell walls biosynthesis. It is taught in J. Amer. Chem. Soc., Vol. 105 (1983), pages 1339—1343 that this interference, which leads to the inhibition of cell growth and, eventually, to the destruction of the cell by lysis, is due to specific binding between these antibiotics and the pentapeptide precursor having a D-Ala-D-Ala residue at the carbonyl terminus (UDP-N-Acetylmuramylpentapeptide). This binding has been exploited for affinity chromatography of murein precursors on vancomycin-sepharose® (FEMS Microbiology Letters, Vol. 9 (1980), pages 215—217). It was recently discovered that also *Teicopolanin** (formerly Teichomycin, U.S. Patent No. 4,239,751) acts the same way by linking to the growing cell-wall of bacteria. More particularly it was found that Teicoplanin, like Vancomycin and Ristocetin A and similar antibiotics links to mucopeptides terminating in D-Ala-D-Ala. By doing so, they probably inhibit the transpeptidase activity and prevent cross-linking of the cell-wall.

Summary of the invention

It has now been found that synthetic analogues of such a ligand portion can be successfully coupled to an insoluble carrier and used to selectively adsorb the target antibiotic (i.e. the antibiotic substance that selectively binds to the ligand). Therefore the present invention is directed to a new insolubilized ligand capable of selectively adsorbing antibiotic substance and to the methods of using the same for binding the target antibiotics.

Thus, one embodiment of the invention is the use of said insolubilized ligand for purifying a target antibiotic from mixture containing it.

It is in fact well known in the art that one of the major problems to be solved in the recovery of an antibiotic substance, for instance from the fermentation broths, is its recovery in a substantially pure form and in a high yield.

Sometimes the contaminants are of the same nature as the substance to be purified, i.e. possess

*INN-International Non-Proprietary Name

chemical- and physical properties very similar to it, and therefore the separation process is very difficult. This purification is oftentimes obtained through numerous and time consuming steps, that in many cases considerably reduce the overall yield. On the contrary, by using the modified matrix of the present invention, a purified solution of a target antibiotic (i.e. an antibiotic which selectively binds to the modified matrix) is obtained often in a single-step procedure. In some instances, however, due to the nature and amount of the contaminants, a higher degree of purity is obtained by submitting the obtained purified solution to a further purification step, by repeating the same operations or by using another purification technic such as liquid-liquid chromatography, e.g. a ion-exchange chromatography, a preparative HPLC, a precipitation by non-solvent and the like.

Therefore, according to the present invention and by using the immobilized ligand herein described, it is possible to selectively absorb a certain antibiotic, separating it from a mixture containing it.

Another embodiment of the invention is the use of the immobilized ligand to screen for antibiotics capable of selectively binding to the ligand. In fact, since the ligand is a synthetic analog of a peptidic chain which is present in the cell-wall of bacteria and it is demonstrated that the antibiotics so far known to link to such peptidic chain possess antimicrobial activity at least against gram-positive bacteria, the immobilized ligand of the invention can be used to screen for antibiotics having this mechanism of action.

Moreover, the fact that the immobilized ligand of the invention selectively adsorbs a certain antibiotic is of considerable importance also for assay methods for said antibiotic, not only in simple aqueous solutions, but also in biological fluids. In fact, the numerous proteinaceous contaminants of a biological fluid, such as urine or blood, are not selectively adsorbed onto the immobilized ligand; only the target antibiotic is. After rinsing with a buffer, the antibiotic can then be eluted, for instance by using another buffer at a different pH, and assayed according to the usual techniques.

Detailed description of the invention

A general view of the coupling of the water-insoluble carriers to the ligands through the introduction of a spacer arm can be found in reference books such as C. R. Lowe and P. G. D. Dean "Affinity Chromatography", John Wiley and Sons Inc. N.Y. 1974 and W. H. Scouten, "Affinity Chromatography", John Wiley and Sons Inc. N.Y. 1981. For example, when a carrier having a hydroxy group

(hereinafter shown as A—OH or 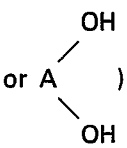 )

is used, the carrier is activated for example with a cyanogen halide (such as cyanogen bromide), a monoepoxide (such as epichlorohydrin), a bioxirane (such as 1,4 - bis(2,3 - epoxypropoxy)butane), a halogenoacetyl halide (such as chloroacetyl chloride) and then the resulting activated carrier is reacted with the ligand which has a free amino group, or the ligand which is linked to a spacer arm having a free amino group (hereinafter both compounds are represented by Ⓛ—$NH_2$); or the above activated carrier is reacted with the ligand having a free hydroxy group or the ligand which is linked to a spacer arm having a free hydroxy group (hereinafter both compounds are represented by Ⓛ—OH).

According to these processes the adsorbents of the following formulae can be obtained:

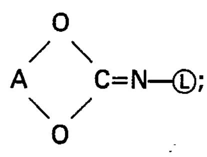

A—OCO—NH—Ⓛ;

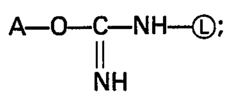

A—O—$CH_2$—CH(OH)$CH_2$NH—Ⓛ;

A—O—$CH_2$CH(OH)$CH_2$—$(CH_2)_m$—$CH_2$CH(OH)$CH_2$NH—Ⓛ;

A—O—$CH_2$CONH—Ⓛ;

A—O—CO$CH_2$NH—Ⓛ;

A—O—$CH_2$CH(OH)$CH_2$—$(CH_2)_m$—$CH_2$CH(OH)$CH_2$—O—Ⓛ;

wherein A and Ⓛ are as above and m is an integer of 1 to 16.

When the water insoluble carrier having an amino group (hereinafter shown as A—$NH_2$) is used,

(1) the carrier is activated with an aliphatic dialdehyde (e.g. glutaraldehyde), the activated carrier is reacted with a free-amino-containing ligand (Ⓛ—$NH_2$) and then the resulting Shiff base is reduced with a reducing agent (e.g. sodium borohydride);

(2) the carrier is reacted with a free carboxy containing ligand (Ⓛ—COOH), i.e. a ligand having a carboxylic function in excess to that of Z group or a ligand into which a spacer arm having said carboxylic group has been introduced;

(3) the carrier is activated with cyanuric halide (cyanuric bromide) and then the resulting activated carrier is reacted with a ligand of formula Ⓛ—$NH_2$;

(4) the carrier is activated with a monoepoxide or bisepoxide and then the resulting activated carrier is reacted with the ligand of formula Ⓛ—$NH_2$ or Ⓛ—OH, wherein Ⓛ—OH and Ⓛ—$NH_2$ are as defined above);

(5) the carrier is diazotized and then reacted with the ligand of formula Ⓛ—$NH_2$.

According to the process, the adsorbents of the following formulae can be obtained:

A—$NHCH_2(CH_2)_mCH_2$—NH—Ⓛ

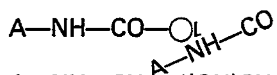

A—NH—$CH_2CH(OH)CH_2NH$—Ⓛ

A—NH—$CH_2$—$CH(OH)CH_2$—O—Ⓛ

A—NH—$CH_2CH(OH)CH_2(CH_2)_mCH_2CH(OH)CH_2NH$—Ⓛ

A—N=N—Ⓛ

wherein Ⓛ, A, and m are as defined above.

When the water-insoluble carrier having a carboxy group (hereinafter A—COOH) is used, the carrier is reacted with a ligand of formula Ⓛ—$NH_2$ to form an acid amide.

According to this process, the adsorbent of the following formula is obtained:

A—CONH—Ⓛ

wherein A and Ⓛ are as above defined.

When the carrier having a halogen atom (hereinafter A-halo) is used, the carrier can be reacted with the ligand of formulae

Ⓛ—$NH_2$ Ⓛ—OH, or Ⓛ—COOH.

According to this process, the adsorbents of the following formulae can be obtained:

A—NH—Ⓛ

A—O—Ⓛ

A—OOC—Ⓛ

wherein A and Ⓛ are as above defined.

The above formulae are not intended to a define stoichiometric balance among the components, since the variations in the ratio of the components, (i.e. carrier, ligand, spacer arm), the way in which they are coupled and the actual sequence of the coupling operations can give polymers and cross-linked modified matrices essentially having the properties defined in the present application—and which are therefore encompassed by the scope of the present invention—that can ultimately differ from the above schematic formulae in stoichiometric or structural aspects.

The water-insoluble carriers of the invention include also a number of commercially available functionalized matrices that can be conveniently used for preparing the modified matrices of the invention according to the techniques herein described or in any case known *per se* to the man skilled in the art.

Examples of said matrices are: Sepharose® (Pharmacia Fine Chemicals, Uppsala, Sweden), Affi-Gel® 202, Affi-Gel 10 and 11 (Bio-Rad Inc., U.S.A.), Eupergit (Röhm Pharma, Weiterstadt, West Germany) and the like.

These functional derivatives are capable of directly linking to the —RCO—D-Ala—Z terminus without any interposed spacers.

In the adsorbent of the present invention the ligand is preferably bonded in an amount of about 2—300 µmol per 1 g (wet form of the adsorbent). "Wet-form" means wet-weight of adsorbent obtained after filtering its aqueous suspension.

Antibiotic containing solution to be contacted with the adsorbent preferably has a pH value of about 7—8.5. However, antibiotic solutions having a pH lower than 7 can also be used, at least in some instances.

When contacting a substrate-containing solution with the adsorbent, either a continuous process using e.g. a column or a batchwise process, using the adsorbent "in bulk", can be employed.

For example, when a column is used the adsorbent may be packed in the column and washed with a salt solution, water, and a buffer solution; then a substrate containing solution is passed through the column to selectively adsorb the target antibiotic on it, the system is then rinsed with the above buffer solution, and finally the adsorbed antibiotic is released from the adsorbent by eluting, for instance, with a buffer of different pH. The so obtained antibiotic is in a purified form, substantially free from many of the original contaminants.

Preferably the first buffer solution has a pH between about 7 and 8.5, while the second buffer (the elution buffer), has a pH higher than 10 and most preferably of about 10 to about 11.5.

On the other hand, when a batchwise process is carried out, a substrate containing solution is added to a suspension of the adsorbent, the resulting mixture is buffered at a pH between 2.5 and 8.5 and preferably at a pH value of 7—8.5 and stirred to selectively absorb the target antibiotic on the adsorbent and then, after having recovered and rinsed the target antibiotic-bearing adsorbent, the target antibiotic is recovered in a purified form by releasing it from the adsorbent by means of a buffer at pH higher than 10, and preferably at a pH between 10 and 11.5. The ratio between the antibiotic-containing solution and the adsorbent to be contacted depends on various parameters such as the total amount of target antibiotic in the solution, the specific adsorbent used, the selected working conditions, in particular the concentration of the target antibiotic and the kind and amount of contaminants.

However these range finding operations are in the range of activity of the skilled technician on the basis of what is disclosed in the present application.

The following examples illustrate the manner in which the invention can be practiced, but, as such, should not be construed as limiting its overall scope.

Example 1
Coupling of the ligand to an activated matrix

A) Preparation of Sephadex® ε-aminocaproyl-D-alanyl-D-alanine

Activated CH-Sepharose 4B (Pharmacia Fine Chemicals) (1 g) is allowed to swell for 15 minutes in 1 mM cold ice hydrochloric acid and washed with the same solution on a glass filter.

The obtained gel (about 3 ml) is mixed with a solution of D-alanyl-D-alanine (30 mg) in 0.5 M sodium chloride and 0.1 M sodium bicarbonate buffer at pH 8.

The mixture is rotated end-over-end for 1 hour at room temperature.

After the coupling reaction is completed, the ligand excess is washed off with the buffer. The unlinked activated groups of the dextrane support are blocked by treating them with 1 M ethanolamine hydrochloride at pH 9 for 1 hour.

Then the Sephadex-ε-aminocaproyl-D-alanyl-D-alanine modified matrix is recovered by filtration and thoroughly washed alternatively with 0.5 M sodium chloride and 0.1 M sodium acetate pH 4, and with 0.5 M sodium chloride and 0.1 M tris(hydroxymethyl)aminomethane buffer pH 8, (four times).

B) Preparation of Sephadex-ε-aminocaproyl-L-alanyl-L-alanine

By operating essentially as above but substituting L-alanyl-L-alanine for D-alanyl-D-alanine, Sephadex-ε-aminocaproyl-L-alanyl-L-alanine is obtained.

C) Preparation of cellulose-poly(D-alanine)

Cellulose (Whatman CF 11, Whatman Inc., U.S.A.), (60 g) is mixed with 1N sodium hydroxyde (400 ml) and epichlorohydrin (400 ml). The mixture is reacted under stirring for about 12 hours. Then, the suspension is filtered and the recovered solid is washed many times with 1N sodium chloride to eliminate the excess of epichlorohydrin, and then with distilled water. The derivatized cellulose is then recovered by filtration yielding about 90 g (wet form).

The above derivatized cellulose (90 g) is suspended in distilled water (300 ml), the pH is adjusted to about 11.5 and 27 g of ε-aminocapronic acid is added thereto.

The mixture is stirred for about 72 hours, keeping the pH at about 11.5. Then after filtering, the recovered solid is thoroughly washed with water yielding about 97 g (wet gel).

N-ethyl-N'-(3-dimethylaminopropyl)carbodiimide hydrochloride (8.5 g) is added to the obtained resin (97 g) suspended in distilled water and the pH is adjusted to about 4.7 with 1N hydrochloric acid. After suitable stirring D-alanine (3.8 g) is added to the mixture and the stirring is continued for about 24 hours, while keeping the pH at about 4.7. Then the cellulose derivative recovered by filtration is thoroughly washed with distilled water, N/10 hydrochloric acid, and again with distilled water.

The excess of unreacted oxirane groups are blocked by stirring this cellulose derivative in 1M ethanolamine at room temperature for 24 hours.

Then the recovered resin is washed with 0.05 M sodium citrate and 0.1 M sodium chloride buffer, pH 8.3. The Cellulose-poly(D-alanine) resin is thus ready for use.

D) Preparation of glass-poly(D-Ala)

3-Aminopropyltriethoxysilane (10 ml) is added to porous glass (GPC-10; Electro-Nucleonics Inc.) (12 g) in methanol/distilled water 1:1 (100 ml). The mixture is gently stirred for 24 hours. Then it is suction-filtered and the glass is thoroughly washed with methanol/distilled water 1:1; then with distilled water alone; 1M sodium chloride, and finally again distilled water.

This activated matrix is suspended in distilled water (100 ml), succinic anhydride (20 g) is added thereto, and the pH is adjusted to about 6. The mixture is stirred at room temperature, while keeping the pH at about 6, for about 2 hours, then it is filtered and the recovered insoluble matrix is washed with distilled water.

The matrix is then resuspended in 50 ml of distilled water and reacted with N-ethyl-N'-(3-dimethylaminopropyl)carbodiimide hydrochloride (4 g), while adjusting the pH to 4.7 by means of 1N hydrochloric acid. After about five minutes, D-alanine (3 g) is added thereto, the pH is kept at about 4.7 and the stirring is continued for 3 hours. Then the mixture is filtered, the recovered resin is thoroughly washed with distilled water, then, sequentially with 1N hydrochloric acid; distilled water; and 0.05 M sodium citrate and 0.1 M sodium chloride, pH 8.3.

E) Preparation of Eupergit C-poly(D-alanine)

Activated acrylamide bearing reactive oxirane groups (Eupergit C; Röhm Pharma, Weitertstadt, West Germany) (5 g) are suspended in 1M potassium phosphate buffer at pH 7.4, and ε-aminocapronic acid is added thereto, while keeping the pH at about 7.4. The suspension is then stirred for about 72 hours, suction-filtered and the recovered matrix is thoroughly washed with water and again filtered.

The wet resin so obtained is resuspended in distilled water (70 ml), and N-ethyl-N'-(3-dimethylaminopropyl)carbodiimide hydrochloride (7.65 g) are added thereto while adjusting the pH to 4.7 by means of 1N hydrochloric acid.

The suspension is stirred for about five minutes and then D-alanine is added thereto, while keeping the pH at about 4.7; the stirring is continued for 24 hours. The modified resin is then recovered by suction filtration and washed sequentially with distilled water, N/1 hydrochloric acid and again distilled water.

The unreacted excess of oxirane groups is inactivated by treating the obtained resin with 10% ethanolamine in 0.01M phosphate buffer at pH 8.9 (150 ml), under gentle stirring for 72 hours. The resin of the title is then recovered by suction filtration followed by sequential washing with distilled water, 1N hydrochloric acid, distilled water, and 0.05M sodium citrate and 0.1M sodium chloride buffer at pH 8.3.

Example 2

Characterization of the foregoing modified matrices for their capacity

A) Determination of the capacity of Sephadex® ε-aminocaproyl-D-alanyl-D-alanine

The following values are obtained by assaying standard samples of known content of Teicoplanin (about 400 μg/ml) by using scalar amount of the modified resin, batchwise:

TABLE 1

| Amount of added resin[a] | % Teicoplanin bound by the resin[b] |
|---|---|
| 0.025 | 46% |
| 0.050 | 88% |
| 0.100 | 97% |
| 0.200 | 98% |
| 0.400 | 98.5% |

[a] ml of wet resin added to 1 ml of Teicoplanin sample

[b] calculated by difference from the content of the sample solutions after filtration.

B) Determination of the capacity of Cellulose-poly(D-alanine)

The following data are obtained by operating as above:

TABLE 2

| Amount of added resin[a)] | % Teicoplanin bound by the resin[b)] |
|---|---|
| 0.2 | 25% |
| 0.4 | 64% |
| 0.6 | 82% |
| 0.8 | 91% |
| 1.0 | 95% |

[a)] ml of wet resin added to 1 ml of Teicoplanin sample
[b)] calculated by difference from the content of the sample solutions after filtration.

C) Determination of the capacity of glass-poly(D-Ala)
The following data are obtained by operating as above (Teicoplanin about 500 µg/ml):

TABLE 3

| Amount of added resin[a)] | % Teicoplanin bound by the resin[b)] |
|---|---|
| 0.005 | 67% |
| 0.010 | 76% |
| 0.025 | 91% |
| 0.050 | 94% |
| 0.100 | 98% |

[a)] ml of wet resin added to 1 ml of Teicoplanin sample
[b)] calculated by difference from the content of the sample solutions after filtration.

D) Determination of the capacity of Eupergit C-poly(D-alanine)
The following data are obtained by operating as above (Teicoplanin content about 500 µg/ml)

TABLE 4

| Amount of added resin[a)] | % Teicoplanin bound by the resin[b)] |
|---|---|
| 0.1 | 62% |
| 0.2 | 85% |
| 0.3 | 90% |
| 0.4 | 92% |
| 0.5 | 95% |
| 0.6 | 99% |

[a)] ml of wet resin added to 1 ml of Teicoplanin sample
[b)] calculated by difference from the content of the sample solutions after filtration.

Example 3
Selective binding of the substrate to the modified matrix
A predetermined amount of modified matrix (Sephadex-ε-aminocaproyl-D-alanyl-D-alanine obtained

according to Example 1A) is added to increasing concentrations of the test antibiotics in 0.15M sodium chloride and 0.05M phosphate buffer pH 7.4. The capped test tubes are rotated end-over-end for 1 hour at room temperature and centrifugated for 5 minutes at 4000 r.p.m.

The concentrations of the free antibiotics in the supernatants are measured by means of known analytical procedures. (U.V. determination at 280 nm, by reference to a $E_{1cm}^{1\%} \approx 51.6$).

Teicoplanin, Vancomycin, Ristocetin A are selectively linked to the matrix while Bacitracin, Actagardin (U.S. Patent 4,022,884), and antibiotic A/16686 (U.S. Patent 4,303,646), do not link to the matrix as they are entirely found in the supernatant. The binding data are elaborated according to G. Scatchard (Ann. N.Y. Acad. Sci. *51,* 660—672 (1949)). The affinity constants as calculated according to the above described experiments are listed in the table below:

TABLE 5

| Antibiotic | Ka($1\times Mole^{-1}$) |
|---|---|
| Teicoplanin | $8.08\times10^{-5}\pm0.13$ |
| Vancomycin | $1.53\times10^{-5}\pm0.36$ |
| Ristocetin A | $4.68\times10^{-5}\pm0.705$ |

The above experiments repeated substituting Sephadex-ε-aminocaproyl-L-alanyl-L-alanine for Sephadex-ε-aminocaproyl-D-alanyl-alanine show that there is *no binding* between the modified matrix and any of the test antibiotics. In particular, the whole amount of Teicoplanin, Vancomycin and Ristocetin A is found in the supernatant.

Example 4
Selective binding of a target antibiotic and its purification

Teicoplanin solutions of different antibiotic contents (purity about 70%) are applied to chromatography columns (0.8×1.5 cm) prepared with the modified matrix obtained according to example 1 A. The column is pre-equilibrated with 0.15 M sodium chloride and 0.05 M sodium phosphate buffer pH 7.4 at room temperature.

The column is then washed with the same buffer and when assayed for Teicoplanin, the collected fractions show a trace content of the antibiotic, while after the elution with 0.15M sodium chloride and sodium phosphate buffer pH 11.5 (flow rate=6 ml/h) the eluted fractions contain about 90—97% of the initial amount of Teicoplanin.

The following table summarizes the experimental data.

TABLE 6

| Experiment No. | Teicoplanin content[a] (mg) S.A.[b] | R.S.[c] | E[d] | % recovery |
|---|---|---|---|---|
| 1 | 28.0 | 0.42 | 27.16 | 97% |
| 2 | 28.0 | 0.42 | 24.4 | 87% |
| 3 | 28.0 | — | 24.9 | 89% |
| 4 | 28.0 | — | 28.76 | 102% |

[a] Teicoplanin is detected by using the HPLC method described in the following Example 5A.
[b] Sample applied to the column.
[c] Rinsing solution (0.15M sodium chloride+0.05M sodium phosphate buffer pH 7.4).
[d] eluate (0.15M sodium chloride+sodium phosphate buffer pH 11.5).

Example 5
Purification of antibiotics by selective binding to the modified matrix of the invention

A) Purification of Teicoplanin with glass-poly(D-alanine)

A fermentation broth obtained by culturing *Actinoplanes teichomyceticus* ATCC 31121 as described in U.S. Patent 4,239,751 (Teicoplanin purity: <5% of the dry material; pH 8.3), is applied to a chromatography column wherein the selective adsorbent is the glass-poly(D-alanine) of example 1D (2.8 cm×11 cm).

The column is prepared with 0.05M sodium citrate, 0.1N sodium chloride buffer at pH 8.3. After

allowing the substrate to be selectively adsorbed to the matrix, the column is rinsed with the above buffer diluted four times in water. The selectively adsorbed substrate is then eluted using 3% ammonia (pH about 11.5; flow rate about 160 ml/h). The eluted fractions are monitored in continuo and assayed for the presence of Teicoplanin (using a Uvicord SVKB spectrophotometer at 280 nm). The fractions containing it are immediately neutralized, pooled and the antibiotic content is determined.

The analytical results, which are reported in Table 7, are obtained by using a HPLC procedure, LCD (C.C.M. System), under the following analytical conditions:

column: RP 18, Brownlee Spheri 5 (Brownlee Lab. Co. Santa Clara, USA): 10 cm, with pre-column
column temperature: 30°C
U.V. detection: 214 nm
eluting mixture: solv. A+solv. B, wherein
solv A: 90% 0.025 M $NaH_2PO_4$+10% $CH_3CN$; pH 6;
solv B: 30% 0.025 M $NaH_2PO_4$+70% $CH_3CN$; pH 6
gradient: T= 0 min, 3% solv. B.
T= 3 min, 3% solv. B
T=30 min, 37% solv. B
flow rate: 1.85 ml/min.

TABLE 7

| Experiment No. | Teicoplanin content | | |
|---|---|---|---|
| | S.A.[a)] | R.S.[b)] | E[c)] |
| 1 | 171 | 6.3% | 94% |
| 2 | 363 | 21.5% | 81% |
| 3 | 363 | 35.8% | 69% |
| 4 | 1676 | 12.7% | 87% |

[a)] total amount of Teicoplanin in the sample (mg)
[b)] % Teicoplanin in the rinsing solutions
[c)] % Teicoplanin detected in the eluate

The purity of these samples, once dried and freed of the added inorganic salts is about 90% (by reference to "pure" standard).

B) Purification of Teicoplanin with cellulose-poly(D-alanine)

By operating substantially as in Example 5A, a Teicoplanin containing fermentation broth is purified in a one-step operation by using cellulose-poly(D-alanine) obtained according to Example 1C. (Size of the column: 5 cm×12 cm); flow rate of the eluent: about 200 ml/h).

TABLE 8

| Experiment No. | Teicoplanin content | | |
|---|---|---|---|
| | S.A.[a)] | R.S.[b)] | E[c)] |
| 1 | 462 mg | 25.8% | 76.8% |
| 2 | 412 mg | 15.7% | 75% |
| 3 | 465 mg | 11.1% | 78% |

[a)] total amount of Teicoplanin in the sample (mg)
[b)] % Teicoplanin in the rinsing solutions
[c)] % Teicoplanin detected in the eluate

C) Purification of Teicoplanin with Eupergit C-poly(D-alanine)

By operating substantially as in the foregoing example, a Teicoplanin-containing fermentation broth is

purified in a one-step operation by using a Eupergit C-poly(D-alanine) resin obtained according to Example 1E (size of the column 2.8×4 cm).

The analytical results reported in the following Table are obtained by operating under the same condition as in the foregoing example.

TABLE 9

| Experiment No. | Teicoplanin content | | |
|---|---|---|---|
| | S.A.[a] | R.S.[b] | E[c] |
| 1 | 100 mg | 32% | 90% |

[a] total amount of Teicoplanin in the sample (mg)
[b] % Teicoplanin in the rinsing solutions
[c] % Teicoplanin detected in the eluate

Example 6
Selective binding of the target antibiotic (dissolved) in a biological fluid

Determination of Teicoplanin in the urines

Each urine sample is adjusted at pH 7.4 by means of 1N hydrochloric acid or 1N sodium hydroxide. In case opalescence or a precipitate forms, it is removed by filtering after centrifugation. Urine samples containing approximately 100 μg of Teicoplanin are diluted to 10 ml with 0.05 M sodium phosphate, 0.15 M sodium chloride, at pH 7.4. The test tubes used are glass tubes treated with 5% dichlorodimethylsilane in toluene for 30 minutes, and completely washed with methanol and anhydrous ethyl ether. The samples containing approximately less than 10 μg/ml of Teicoplanin are kept undiluted. Following the same procedure, 10 ml samples containing 10 μg/ml of Teicoplanin are prepared (standard solutions). A homogeneous suspension (1:1) of CH-Sepharose 4B (Pharmacia Fine Chemicals) prepared as described in Example 1A, in the same buffer as above (0.5 ml), is added to each sample. The mixture is gently stirred for about 1 hour. Then the samples are centrifuged (5 minutes×4000 r.p.m.) and, after having discarded the supernatant, resuspended in 10 ml of buffer. Each sample is again centrifuged as above and the supernatant is again discarded leaving the resin in about 0.5 ml of solution.

The antibiotic selectively adsorbed to the resin is eluted by treating with 0.05 sodium phosphate buffer at pH 11.6, using 50 μg/ml orcinol as the internal standard. The mixture is gently stirred for about 30 minutes, then the resin is eliminated by centrifugation and the supernatant is neutralized with 1N HCl (50 ml).

The analysis is carried out according to the HPLC method already described, using a solution of Teicoplanin of the same content as the standard solution to serve as the blank (i.e. a sample which is assayed in parallel with the other samples, but which is not added to the resin).

The following Tables summarize the experimental data:

TABLE 10

| Experiment No. | % Recovery of Teicoplanin from urines |
|---|---|
| 1 | 73.8 |
| 2 | 72.5 |
| 3 | 74 |
| 4 | 78 |
| average recovery=74.5% | |

TABLE 11

| Experiment No. | % Recovery of Teicoplanin from the standard solution |
|---|---|
| 1 | 76.5 |
| 2 | 71.6 |
| 3 | 72.8 |
| 4 | 88.1 |
| 5 | 75.4 |
| 6 | 81.4 |
| average recovery =77.6% | |

## Claims

1. New modified matrix of the formula

A—B—R—CO—D-Ala—Z

wherein A represents a water-insoluble carrier, B represents a bond, a spacer arm, or a polyaminoacidic residue, R represents a straight or branched ($C_1$—$C_{12}$)alkylene substituted amino or oxy group of formula:

—HN—($C_1$—$C_{12}$)alkylene or —O—($C_1$—$C_{12}$)alkylene

respectively, or R—CO represents a L-lysinyl residue, or a bond or a —$OC(CH_2)_nCO$— group wherein n is an integer from 1 to 6 inclusive, and preferably 2, 3 or 4; Z represents an aminoacidic residue selected from D-alanyl, glycinyl, and D-leucinyl; and D-Ala represents a D-alanyl group; with the proviso that when R—CO is a bond B is a group having a carboxy terminal function that links to the amino function of the D-alanine, or in the case also B is a bond, A is a water-insoluble carrier having a carboxy terminal function that links to the amino function of the D-alanine through a peptidic bond.

2. A modified matrix as in claim 1 wherein R is a straight or branched ($C_2$—$C_6$)alkylene substituted amino or oxy group of formula:

—HN—($C_2$—$C_6$)alkylene or —O—($C_2$—$C_6$)alkylene,

respectively.

3. A modified matrix as in claim 1 wherein the water-insoluble carrier is selected from those bearing hydroxy, amine or carbonyl groups or halogen (chloro, bromo) atoms.

4. A modified matrix as in claim 1 wherein the water-insoluble carrier is selected from polysaccharides, hydroxyalkylpolystyrene, polyvinyl alcohols, aminoalkylpolysaccharides, p-aminobenzylpolysaccharides, chitosan, aminoalkylpolystyrenes, polyacrylamides, aminopolyacrylamides, aminoalkyl porous glasses, carboxyalkylpolysaccharides, carboxyalkylacrylamides, and halogenoalkylpolystyrenes.

5. A modified matrix as in claim 1 wherein the water-insoluble carrier is selected from cellulose, agarose, cross-linked dextran, hydroxyalkylated styrene - divinylbenzene copolymer, aminoalkylcellulose, aminoalkylagarose, p-aminobenzylcellulose, p-aminobenzylagarose, aminoalkylated styrene - divinylbenzene copolymer, aminoethylpolyacrylamide, aminopropyl - porous glass, carboxyalkylagarose, carboxyalkylcellulose, carboxyalkyl - cross - linked dextran, carboxymethylpolyacrylamide, acrylic acid - divinylbenzene copolymer, chloromethylated styrene - divinylbenzene copolymer.

6. A modified matrix as in claim 1 wherein the water insoluble carrier is selected from cellulose, carboxymethylcellulose, glass, controlled pore glass, cross-linked dextrans, agarose, and carboxymethyl agarose.

7. A modified matrix as in claim 1, wherein the symbol B represents a bond.

8. A modified matrix as in claim 1, wherein B is selected from the residues of the formulas:

—HN—$(CH_2)_m$—NH— —$OC(CH_2)_mCO$— —$HN(CH_2)_mCO$— or —$HN(CH_2)_mO$—

wherein m represents an integer from 1 to 12.

9. A modified matrix as in claim 1, wherein B represents —HN—$(CH_2)_m$—CO—, wherein m is an integer of 2 to 6 inclusive.

10. A modified matrix as in claim 1, wherein R—CO—D-Ala—Z represents a poly(D-alanine) chain of about 2 to 15 residues.

11. A modified matrix as in claim 1, wherein Z is D-alanine.

12. A modified matrix as in claim 1, wherein R—CO represents a bond and B is a group having a carboxy terminal function that links the amino function of the D-alanine through a peptidic bond.

13. A modified matrix as in claim 1, wherein R—CO and B both represent a bond, and A is a water-insoluble carrier having a carboxy terminal function that links the amino function of the D-alanine through a peptidic bond.

14. A modified matrix as in claim 1, for use in purifying substances that are selectively adsorbed by the said modified matrix.

15. A modified matrix as in claim 1, for purifying Teicoplanin, Vancomycin, Ristocetin A and the like antibiotics that selectively link to mucopeptides having a D-Ala-D-Ala group at the —COOH terminus.

16. Use of a matrix as in claim 1, for purifying substances that are selectively adsorbed by said modified matrix.

17. A method for purifying Teicoplanin, Vancomycin, Ristocetin A or derivatives thereof that selectively bind to the modified matrix of claim 1 which comprises:

a) adsorbing the target antibiotic onto such matrix

b) rinsing with a first buffer solution that does not elute the target antibiotic from the matrix

c) eluting the target antibiotic with a second buffer solution.

18. A method as in claim 17 wherein the first buffer solution is a solution having a pH value lower than 8.5.

19. A method as in claim 17 wherein the first buffer solution is a buffer solution having a pH value between 7 and 8.5, both inclusive.

20. A method as in claim 17 wherein the second buffer solution has a pH value higher than 10.

21. A method as in claim 17 wherein the second buffer solution has a pH value between 10 and 11.5, both inclusive.

22. A method for screening for antibiotic substances that link to bacterial cell-wall, and in particular to its mucopeptidic constituents which terminate in D-Ala-D-Ala which comprises:

a) contacting a mixture (solution, fermentation broth, etc.) which can contain said antibiotic substance with the modified matrix of claim 1

b) allowing the substrate (if present) to be selectively adsorbed to the matrix

c) rinsing with a rinsing solution that does not significatively elute the selectively adsorbed substrate, (if any)

d) eluting the selectively adsorbed substrate, (if any), and recovering it according to known *per se* techniques.

23. A method as in claim 22 wherein the rinsing solution is a buffer solution having a pH value lower than 8.5.

24. A method as in claim 22 wherein the rinsing solution is a buffer solution having a pH value between 7 and 8.5, both inclusive.

25. A method as in claim 22 wherein the eluting solution is a buffer solution having a pH value higher than 10.

26. A method as in claim 22 wherein the eluting solution is a buffer solution having a pH value between 10 and 11.5, both inclusive.

27. A modified matrix as in claim 1 wherein the water-insoluble carrier A is selected from carboxymethylcellulose, celluloe, porous glass, cross-linked dextrans, agarose, carboxymethylagarose, the ligand RCO—D-Ala—Z is a poly(D-Ala) residue of 2 to 15 residues and wherein Z is D-Ala and the bond between the ligand and the carrier is obtained by means of cyanogen bromide, epichlorohydrin, 1,4 - bis - (2,3 - diepoxypropoxy)butane or 3 - aminopropyltriethoxy silane.

**Patentansprüche**

1. Neue modifizierte Matrix der Formel

A—B—R—CO—D-Ala—Z

in der A einen wasserunlöslichen Träger darstellt, B eine Bindung, einen Abstandsarm oder einer Polyaminosäurerest bedeutet, R für eine geradkettig oder verzweigt $(C_1—C_{12})$-alkylen-substituierte Amino- oder Oxygruppe der Formel:

—HN—$(C_1—C_{12})$-Alkylen oder —O—$(C_1—C_{12}$-Alkylen

steht oder R—CO einen L-Lysinyl-Rest oder eine Bindung oder eine —OC$(CH_2)_n$CO— Gruppe bedeutet, in der n eine ganze Zahl von 1 bis 6 einschließlich, vorzugsweise 2, 3 oder 4, ist; Z einen Aminosäurerest,

ausgewählt aus D-Alanyl, Glycinyl und D-Leucinyl, bedeutet; und D-Ala für eine D-Alanyl-Gruppe steht mit der Maßgabe, daß, wenn R—CO eine Bildung bedeutet, B eine Gruppe mit einer endständigen Carboxygruppe ist, die mit der Aminogruppe des D-Alanins eine Bildung eingeht, oder, wenn auch B eine Bindung bedeutet, A ein wasserunlöslicher Träger mit einer endständigen Carboxygruppe ist, der über eine Peptidbindung mit der Aminogruppe des D-Alanins eine Bildung eingeht.

2. Modifizierte Matrix nach Anspruch 1, dadurch gekennzeichnet, daß R eine geradkettig oder verzweigt ($C_2$—$C_6$)-alkylen-substituierte Amino- oder Oxygruppe der Formel

—HN—($C_2$—$C_6$)-Alkylen oder —O—($C_2$—$C_6$)-Alkylen

ist.

3. Modifizierte Matrix nach Anspruch 1, dadurch gekennzeichnet, daß der wasserunlösliche Träger ausgewählt wird aus den Trägern, die Hydroxy-, Amin- oder Carbonylgruppen oder Halogen- (Chlor-, Brom-) Atome tragen.

4. Modifizierte Matrix nach Anspruch 1, dadurch gekennzeichnet, daß der wasserunlösliche Träger ausgewählt wird aus Polysacchariden, Hydroxyalkylpolystyrolen, Polyvinylalkoholen, Aminoalkylpolysacchriden, p-Aminobenzylpolysacchariden, Chitosan, Aminoalkylpolystyrolen, Polyacrylamiden, Aminopolyacrylamiden, aminoalkyl - poröse Gläser, Carboxyalkylpolysacchariden, Carboxyalkylacrylamiden und Halogenalkylpolystyrolen.

5. Modifizierte Matrix nach Anspruch 1, dadurch gekennzeichnet, daß der wasserunlösliche Träger ausgewählt wird aus Cellulose, Agarose, vernetztem Dextran, hydroxyalkyliertem Styrol - Divinylbenzol - Copolymer, Aminoalkylcellulose, Aminoalkylagarose, p-Aminobenzylcellulose, p-Aminobenzylagarose, aminoalkyliertem Styrol - Divinylbenzol - Copolymer, Aminoethylpolyacrylamid, aminopropyl - porösem Glas, Carboxyalkylagarose, Carboxyalkylcellulose, carboxyalkyl - vernetztem Dextran, Carboxymethylpolyacrylamid, Acrylsäure - Divinylbenzol - Copolymer, chlormethyliertem Styrol - Divinylbenzol - Copolymer.

6. Modifizierte Matrix nach Anspruch 1, dadurch gekennzeichnet, daß der wasserunlösliche Träger ausgewählt wird aus Cellulose, Carboxymethylcellulose, Glas, Glas mit geregelter Porengröße, vernetzten Dextranen, Agarose und Carboxymethylagarose.

7. Modifizierte Matrix nach Anspruch 1, dadurch gekennzeichnet, daß das Symbol B eine Bindung bedeutet.

8. Modifizierte Matrix nach Anspruch 1, dadurch gekennzeichnet, daß B ausgewählt wird aus den Resten der Formeln:

—HN—$(CH_2)_m$—NH— —$OC(CH_2)_mCO$— —$HN(CH_2)_mCO$— oder —$HN(CH_2)_mO$—

in denen m eine ganze Zahl von 1 bis 12 bedeutet.

9. Modifizierte Matrix nach Anspruch 1, dadurch gekennzeichnet, daß B für —HN—$(CH_2)_m$—CO— steht, worin m eine ganze Zahl von 2 bis 6 einschließlich ist.

10. Modifizierte Matrix nach Anspruch 1, dadurch gekennzeichnet, daß R—CO—D-Ala—Z eine Poly(D-Alanin)-Kette mit etwa 2 bis 15 Resten bedeutet.

11. Modifizierte Matrix nach Anspruch 1, dadurch gekennzeichnet, daß das Z für D-Alanin steht.

12. Modifizierte Matrix nach Anspruch 1, dadurch gekennzeichnet, daß R—CO eine Bindung bedeutet und B für eine Gruppe mit einer endständigen Carboxygruppe steht, die an die Aminogruppe des D-Alanins über eine Peptidbindung gebunden ist.

13. Modifizierte Matrix nach Anspruch 1, dadurch gekennzeichnet, daß R—CO und B beide eine Bindung bedeuten und A für einen wasserunlöslichen Träger steht, der eine endständige Carboxygruppe aufweist, die mit der Aminogruppe des D-Alanins eine Peptidbindung eingeht.

14. Modifizierte Matrix nach Anspruch 1 zur Verwendung beim Reinigen von Substanzen, die selektiv von der modifizierten Matrix adsorbiert werden.

15. Modifizierte Matrix nach Anspruch 1 zum Reinigen von Teicoplanin, Vancomycin, Ristocetin A und ähnlichen Antibiotika, die selektiv an Mucopeptide mit einer D-Ala-D-Ala-Gruppe am —COOH-Ende gebunden werden.

16. Verwendung einer Matrix nach Anspruch 1 zum Reinigen von Substanzen, die selektiv von der modifizierten Matrix adsorbiert werden.

17. Verfahren zum Reinigen von Teicoplanin, Vancomycin, Ristocetin A oder Derivaten davon, die selektiv an die modifizierte Matrix nach Anspruch 1 gebunden werden, dadurch gekennzeichnet, daß

a) das Zielantibiotikum auf diese Matrix adsorbiert wird,

b) mit einer ersten Pufferlösung gespült wird, die das Zielantibiotikum nicht von der Matrix eluiert,

c) das Zielantibiotikum mit einer zweiten Pufferlösung eluiert wird.

18. Verfahren nach Anspruch 17, dadurch gekennzeichnet, daß die erste Pufferlösung eine Lösung mit einem pH-Wert unter 8,5 ist.

19. Verfahren nach Anspruch 17, dadurch gekennzeichnet, daß die erste Pufferlösung eine Pufferlösung mit einem pH-Wert zwischen 7 und 8,5 ist, beide Grenzwerte eingeschlossen.

20. Verfahren nach Anspruch 17, dadurch gekennzeichnet, daß die zweite Pufferlösung einen pH-Wert über 10 aufweist.

21. Verfahren nach Anspruch 17, dadurch gekennzeichnet, daß die zweite Pufferlösung einen pH-Wert zwischen 10 und 11,5, jeweils eingeschlossen, aufweist.

22. Verfahren zum Nachweis von antibiotischen Substanzen, die an Bakterienzellwände gebunden sind, insbesondere an deren Mucopeptidbestandteile, die in D-Ala-D-Ala enden, dadurch gekennzeichnet, daß man:

a) ein Gemisch (Lösung, Fermentationsbrühe usw.), das die antibiotische Substanz enthalten kann, mit der modifizierten Matrix nach Anspruch 1 in Berührung bringt,

b) das Substrat, wenn vorhanden, selektiv von der Matrix adsorbieren läßt,

c) mit einer Spüllösung spült, die nicht merklich das selektiv adsorbierte Substrat (wenn vorhanden) eluiert,

d) das selektiv adsorbierte Substrat (wenn vorhanden) eluiert und in an sich bekannter Weise gewinnt.

23. Verfahren nach Anspruch 22, dadurch gekennzeichnet, daß die Spüllösung eine Pufferlösung mit einem pH-Wert unter 8,5 ist.

24. Verfahren nach Anspruch 22, dadurch gekennzeichnet, daß die Spüllösung eine Pufferlösung mit einem pH-Wert zwischen 7 und 8,5, beide Werte eingeschlossen, ist.

25. Verfahren nach Anspruch 22, dadurch gekennzeichnet, daß die Elutionslösung eine Pufferlösung mit einem pH-Wert über 10 ist.

26. Verfahren nach Anspruch 22, dadurch gekennzeichnet, daß die Elutionslösung eine Pufferlösung mit einem pH-Wert zwischen 10 und 11,5, jeweils eingeschlossen, ist.

27. Modifizierte Matrix nach Anspruch 1, dadurch gekennzeichnet, daß der wasserunlösliche Träger A ausgewählt wird aus Carboxymethylcellulose, Cellulose, porösem Glas, vernetzten Dextranen, Agarose, Carboxymethylagarose, daß der Ligand RCO-D-Ala-Z ein Poly(D-Ala)-Rest mit 2 bis 15 Einheiten ist und worin Z D-Ala bedeutet und die Bindung zwischen Ligand und Träger mit Hilfe von Bromcyan, Epichlorhydrin, 1,4-Bis-2,3-diepoxypropoxy)-butan oder 3-Aminopropyltriethoxysilan erzielt worden ist.

**Revendications**

1. Nouvelle matrice modifiée, de formule

A—B—R—CO—D-Ala—Z

dans laquelle A représente un support insoluble dans l'eau, B représente une liaison directe, un groupe pont (en anglais: spacer arm), ou un résidu polyaminoacide, R représente un groupe amino ou oxy, substitué par un radical alkylène en $C_1$—$C_{12}$ à chaîne droite ou ramifiée, ayant respectivement les formules suivantes:

—HN—(alkylène en $C_1$—$C_{12}$) ou —O—(alkylène en $C_1$—$C_{12}$),

ou bien R—CO représente un résidu L-lysinyle, ou une liaison directe ou un groupe $—OC(CH_2)_nCO—$ où n est un entier de 1 à 6 inclusivement, et de préférence égal à 2, 3 ou 4; Z représente un résidu acide aminé choisi parmi les résidus D-alanyle, glycinyle et D-leucinyle; et D-Ala représente un groupe D-alanyle, à la condition que, quand R—CO est une liaison directe, B soit un groupe comportant une fonction carboxy-terminal qui se fixe à la fonction amino de la D-alanine, ou bien, si B est lui aussi une liaison directe, A soit un support insoluble dans l'eau comportant une fonction carboxy-terminal qui se fixe à la fonction amino de la D-alanine par l'intermédiaire d'une liaison peptidique.

2. Matrice modifiée selon la revendication 1, dans laquelle R est un groupe amino ou oxy, substitué par un radical alkylène en $C_2$—$C_6$ à chaîne droite ou ramifiée, ayant respectivement les formules suivantes:

—HN—(alkylène en $C_2$—$C_6$) ou —O—(alkylène en $C_2$—$C_6$).

3. Matrice modifiée selon la revendication 1, dans laquelle le support insoluble dans l'eau est choisi parmi ceux comportant des groupes hydroxy, amino ou carbonyle ou des atomes d'halogène (chloro, bromo).

4. Matrice modifiée selon la revendication 1, dans laquelle le support insoluble dans l'eau est choisi parmi les polysaccharides, les polystyrènes hydroxyalkylés, les poly(alcools vinyliques), les aminoalkylpolysaccharides, les p-aminobenzylpolysaccharides, le chitosane, les polystyrèneaminoalkylés, les polyacrylamides, les aminopolyacrylamides, les verres poreux aminoalkylés, les carboxyalkylpolysaccharides, les carboxyalkylacrylamides et les polystyrènehalogénoalkylés.

5. Matrice modifiée selon la revendication 1, dans laquelle le support insoluble dans l'eau est choisi parmi la cellulose, l'agarose, le dextrane réticulé, les copolymères styrène - divinylbenzènehydroxyalkylés, les aminoalkylcelluloses, les aminoalkylagaroses, la p-aminobenzylcellulose, la p-aminobenzylagarose, les copolymères styrène - vinylbenzèneaminoalkylés, l'aminoéthylpolyacrylamide, le verre poreux amino-

propylé, les carboxyalkylagaroses, les carboxyalkylcelluloses, les dextranes réticulés carboxyalkylés, le carboxyméthylpolyacrylamide, le copolymère acide acrylique - divinylbenzène, le copolymère styrène - divinylbenzènechlorométhylé.

6. Matrice modifiée selon la revendication 1, dans laquelle le support insoluble dans l'eau est choisi parmi la cellulose, la carboxyméthylcellulose, le verre, le verre à pores contrôlés, les dextranes réticulés, l'agarose et la carboxyméthylagarose.

7. Matrice modifiée selon la revendication 1, dans laquelle le symbole B représente une liaison directe.

8. Matrice modifiée selon la revendication 1, dans laquelle B est choisi parmi les résidus de formules:

$$—NH—(CH_2)_m—NH— \quad —OC(CH_2)_mCO— \quad —HN(CH_2)_mCO— \quad \text{ou} \quad —HN(CH_2)_mO—$$

dans lesquelles m représente un entier de 1 à 12.

9. Matrice modifiée selon la revendication 1, dans laquelle B représente $—HN—(CH_2)_m—CO—$, où m est un entier de 2 à 6 inclusivement.

10. Matrice modifiée selon la revendication 1, dans laquelle R—CO—D-Ala—Z représente une chaîne poly(D-alanine) constituée d'environ 2 à 15 résidus.

11. Matrice modifiée selon la revendication 1, dans laquelle Z est la D-alanine.

12. Matrice modifiée selon la revendication 1, dans laquelle R—CO représente une liaison directe et B est un groupe comportant une fonction carboxy-terminal qui se fixe à la fonction amino de la D-alanine par l'intermédiaire d'une liaison peptidique.

13. Matrice modifiée selon la revendication 1, dans laquelle R—CO et B représentent chacun une liaison directe, et A est un support insoluble dans l'eau comportant une fonction carboxy-terminal qui se fixe à la fonction amino de la D-alanine par l'intermédiaire d'une liaison peptidique.

14. Matrice modifiée selon la revendication 1, pour être utilisée à la purification de substances qui sont sélectivement adsorbées par ladite matrice modifiée.

15. Matrice modifiée selon la revendication 1, pour purifier la téicoplanine, la vancomycine, la ristocétine A et les antibiotiques analogues qui se fixent sélectivement sur des mucopeptides comportant un groupe D-Ala-D-Ala sur le site terminal —COOH.

16. Utilisation d'une matrice selon la revendication 1, pour purifier des substances qui sont sélectivement adsorbées par ladite matrice modifiée.

17. Procédé pour purifier la téicoplanine, la vancomycine, la ristocétine A ou leurs dérivés, qui se fixent sélectivement sur la matrice modifiée de la revendication 1, qui consiste à:

a) faire adsorber l'antibiotique-cible par la matrice,

b) rincer avec une première solution tampon qui n'élue pas l-antibiotique-cible de la matrice,

c) éluer l'antibiotique-cible à l'aide d'une deuxième solution tampon.

18. Procédé selon la revendication 17, dans lequel la première solution tampon est une solution ayant une valeur de pH inférieure à 8,5.

19. Procédé selon la revendication 17, dans lequel la première solution tampon est une solution tampon ayant une valeur de pH comprise entre 7 et 8,5, limites comprises.

20. Procédé selon la revendication 17, dans lequel la deuxième solution tampon a une valeur de pH supérieure à 10.

21. Procédé selon la revendication 17, dans lequel la deuxième solution tampon a une valeur de pH comprise entre 10 et 11,5, limites comprises.

22. Procédé pour trier des substances antibiotiques qui se fixent à la paroi cellulaire des bactéries, et en particulier à ses constituants mucopeptidiques comportant D-Ala-D-Ala en position terminale, consistant à:

a) mettre en contact un mélange (solution, bouillon de fermentation, etc.), qui peut contenir ladite substance antibiotique, avec la matrice modifiée de la revendication 1,

b) permettre au substrat (s'il est présent) d'être sélectivement adsorbé par la matrice,

c) rincer à l'aide d'une solution de rinçage qui n'élue pas d'une manière significative l'éventuel substrat sélectivement adsorbé,

d) éluer l'éventuel substrat sélectivement adsorbé, et le récupérer par des techniques connues en soi.

23. Procédé selon la revendication 22, dans lequel la solution de rinçage est une solution tampon ayant une valeur de pH inférieure à 8,5.

24. Procédé selon la revendication 22, dans lequel la solution de rinçage est une solution tampon ayant une valeur de pH comprise entre 7 et 8,5, limites incluses.

25. Procédé selon la revendication 22, dans lequel la solution d'élution est une solution tampon ayant une valeur de pH supérieure à 10.

26. Procédé selon la revendication 22, dans lequel la solution d'élution est une solution tampon ayant une valeur de pH comprise entre 10 et 11,5, limites comprises.

27. Matrice modifiée selon la revendication 1, dans laquelle le support insoluble dans l'eau A est choisi parmi la carboxyméthylcellulose, la cellulose, le verre poreux, les dextranes réticulés, l'agarose, la carboxyméthylagarose, le ligand R-CO—D-Ala—Z est un résidu poly(D-Ala) constitué de 2 à 15 résidus, et dans laquelle Z est D-Ala, la liaison entre le ligand et le support étant obtenue au moyen de bromure de cyanogène, d'épichlorhydrine, de 1,4 - bis - (2,3 - diépoxypropoxy)-butane ou de 3 - aminopropyl-triéthoxysilane.